# EUROPEAN PATENT SPECIFICATION

(11) **EP 2 514 425 B1**
(45) Date of publication and mention of the grant of the patent: **17.09.2014**
(21) Application number: 10837406.7
(22) Date of filing: 19.11.2010
(51) Int. Cl.: A61K 31/575, A23L 1/30, A23L 2/52, A61K 31/58, A61P 3/08, A61P 29/00, A61P 35/00

(54) **COMPOSITION CONTAINING PROTOPANAXATRIOL AND PROTOPANAXADIOL**
ZUSAMMENSETZUNG MIT PROTOPANAXATRIOL UND PROTOPANAXADIOL
COMPOSITION CONTENANT DU PROTOPANAXATRIOL ET DU PROTOPANAXADIOL

(30) Priority: 14.12.2009 JP 2009282764
(43) Date of publication of application: 24.10.2012
(73) Proprietor: Lion Corporation, Tokyo 130-8644 (JP)
(72) Inventor: IWASAKI, Hideaki, Tokyo 130-8644 (JP); NOMURA, Mitsuru, Tokyo 130-8644 (JP); SUZUKI, Naho, Tokyo 130-8644 (JP); KAMBAYASHI, Hiroaki, Tokyo 130-8644 (JP); KITAMURA, Kumiko, Tokyo 130-8644 (JP)
(74) Representative: Graf von Stosch, Andreas
(86) International application number: PCT/JP2010/070708
(87) International publication number: WO 2011/074377

(56) References cited:
- WO-A1-2010/029915
- CN-A- 1 417 224
- CN-A- 1 850 099
- CN-A- 1 962 681
- KR-A- 20070 109 282
- KR-A- 20070 109 282
- CUI J F ET AL: "Analysis of Ginsenosides by Chromatography and Mass Spectrometry: Release of 20 S-Protopanaxadiol and 20 S-Protopanaxatriol for Quantitation", ANALYTICAL BIOCHEMISTRY, ACADEMIC PRESS INC, NEW YORK, vol. 210, no. 2, 1 May 1993 (1993-05-01), pages 411-417, XP024764016, ISSN: 0003-2697, DOI: 10.1006/ABIO.1993.1215 [retrieved on 1993-05-01]
- SHIBATA, S. ET AL.: 'Chemical studies on oriental plant drugs. XIV. Protopanaxadlol, a genuine sapogenin of Ginseng saponins' CHEM PHARM BULL vol. 14, no. 6, 1966, pages 595 - 600
- ZHAO, Y. ET AL.: 'Rare constituents with anti-cancer activity from hydrolytic products in saponins of Panax quinquefolium stems and leaves' CHINESE TRADITIONAL AND HERBAL DRUGS vol. 39, no. 9, 2008, pages 1291 - 1294
- HIAI, S. ET AL.: 'A color reaction of panaxadiol with vanillin and sulfuric acid' PLANTA MEDICA vol. 28, no. 2, 1975, pages 131 - 138, XP008159133

## Description

### Technical Field

The present invention relates to a composition containing at least one of protopanaxatriol and protopanaxadiol for medical use in pharmaceutical drugs and to the use of such a composition for the preparation of food or beverage, dietary supplements or of pharmaceutical drugs.

### Background Art

Protopanaxatriol (PPT) and protopanaxadiol (PPD) are known to have various physiological activities such as an anti-cancer effect (see PTLs 1 and 2), an anti-inflammatory effect on skin diseases (see PTL 3), an effect of suppressing excretion of urinary albumin (see PTL 4) and an effect of activating PPARγ which regulates the expression of genes playing important roles in fat metabolism and sugar metabolism (see PTL 5).

Protopanaxatriol (PPT) and protopanaxadiol (PPD) are aglycons remaining after removal of the sugar moiety from saponin (glycoside) (ginsenoside) contained in ginseng. They are compounds belonging to dammarane-type triterpenes.

Although protopanaxatriol (PPT) and protopanaxadiol (PPD) are white powder and insoluble to water, their solubility can be increased by the addition of an organic solvent.

The effect of saponin differs among individuals since saponin has low absorbability into bodies and its sugar is decomposed by intestinal bacteria before absorption. Meanwhile, such aglycons as protopanaxatriol (PPT) and protopanaxadiol (PPD) do not have sugar moieties, and thus can be expected to minimize the differences among individuals.

A publication by Cui et al. (Analytical Biochemistry, Academic Press Inc., New York, vol. 210, no. 2, 1 May 1993 (1993-05-01), pages 411-417, XP024764016) describes the analysis of ginsenosides by chromatography and mass spectrometry upon acid or alkaline hydrolyses. Panaxatriol (PT), protopanaxatriol (PPT), panaxadiol (PD) and protopanaxadiol (PPD) were described in the analysis samples.

However, protopanaxatriol (PPT) and protopanaxadiol (PPD) each have an unstable structure and disadvantageously, they are decomposed minute by minute in a liquid system, especially in a low-pH system. Even in the powder form, they are progressively decomposed day by day at a temperature equal to or higher than ambient temperature. As described above, there is a problem that their stability is low.

Accordingly, at present, keen demand has arisen for provision of a more stable composition containing a large amount of at least one of protopanaxatriol (PPT) and protopanaxadiol (PPD) having physiological activities such as an anti-cancer effect, an anti-inflammatory effect and a sugar metabolism-regulating effect, and a highly safe food or beverage containing the composition.

### Citation List

### Patent Literature

- PTL 1:: Japanese Patent Application Laid-Open (JP-A) No. 2005-504799
- PTL 2:: JP-A No. 58-57399
- PTL 3:: JP-A No. 2007-008896
- PTL 4:: JP-A No. 10-212296
- PTL 5:: Korean Patent Application Laid-Open No. 10-2006-0131012

### Summary of Invention

### Technical Problem

The present invention aims to solve the above existing problems and achieve the following objects. Specifically, an object of the present invention is to provide: a more stable composition containing a large amount of at least one of protopanaxatriol (PPT) and protopanaxadiol (PPD) having physiological activities such as an anti-cancer effect, an anti-inflammatory effect and a sugar metabolism-regulating effect; use of such a composition for a highly safe food or beverage.

### Solution to Problem

The present inventors conducted extensive studies to solve the above existing problems and have obtained the following findings. That is, they have found that mixing panaxatriol (PT) and protopanaxatriol (PPT) at a predetermined ratio improves stability of protopanaxatriol (PPT) and that mixing panaxadiol (PD) and protopanaxadiol (PPD) at a predetermined ratio improves stability of protopanaxadiol (PPD). The present invention has been accomplished on the basis of these findings.

The present invention is based on the above findings obtained by the present inventors. Means for solving the problems are as follows.
<1> A composition for use in pharmaceutical drugs comprising:
   at least one mixture selected from the group consisting of a mixture of (A) panaxatriol and (B) protopanaxatriol and a mixture of (C) panaxadiol and (D) protopanaxadiol,
   wherein a ratio (A)/(B) of a mass of the (A) panaxatriol to a mass of the (B) protopanaxatriol is 1 or greater, and a ratio (C)/(D) of a mass of the (C) panaxadiol to a mass of the (D) protopanaxadiol is 1 or greater.
<2> Use of a composition comprising:
   at least one mixture selected from the group consisting of a mixture of (A) panaxatriol and (B) protopanaxatriol and a mixture of (C) panaxadiol and (D) protopanaxadiol,
   wherein a ratio (A)/(B) of a mass of the A) panaxatriol to a mass of the (B) protopanaxatriol is 1 or greater, and a ratio (C)/(D) of a mass of the (C) panaxadiol to a mass of the (D) protopanaxadiol is 1 or greater
      for the preparation of food or beverage, dietary supplements or of pharmaceutical drugs.
<3> Use of a composition according to <2>, wherein the composition is a food or beverage.

### Advantageous Effects of Invention

The present invention can provide: a more stable composition containing a large amount of at least one of protopanaxatriol (PPT) and protopanaxadiol (PPD) having physiological activities such as an anti-cancer effect, an anti-inflammatory effect and a sugar metabolism-regulating effect, which is used in a highly safe food, in dietary supplements or in pharmaceutical drugs. These can solve the above existing problems and achieve the above objects.

### Description of Embodiments

### (Composition)

A composition for medical use according to the present invention includes at least one mixture selected from the group consisting of a mixture of (A) panaxatriol and (B) protopanaxatriol and a mixture of (C) panaxadiol and (D) protopanaxadiol; and, if necessary, further includes other ingredients. Such a composition is used according to the invention for the preparation of food or beverage, dietary supplements or of pharmaceutical drugs as well.

### <Ingredient (A)>

Panaxatriol (PT) (Ingredient (A)) is a compound having a structure expressed by the following Structural Formula (1). Panaxatriol (PT) is a compound belonging to dammarane-type triterpenes. It is an aglycon formed after the sugar moiety has been removed from a plant-origin saponin (glycoside) and then the side chain has been ring-closed.

The method for obtaining Ingredient (A) is not particularly limited and may be appropriately selected depending on the intended purpose. Examples thereof include a method in which Ingredient (A) is extracted from plants, a method in which Ingredient (A) is obtained through synthesis, and a method in which commercially available products of Ingredient (A) are used.

Incorporation of Ingredient (A) into the composition can advantageously stabilize Ingredient (B).

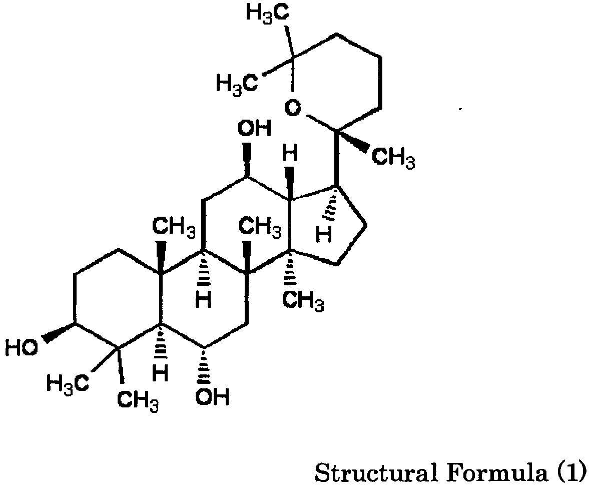

### <Ingredient (B)>

Protopanaxatriol (PPT) (Ingredient (B)) is a compound having a structure expressed by the following Structural Formula (2).

Protopanaxatriol (PPT) is a compound belonging to dammarane-type triterpenes. It is an aglycon formed after the sugar moiety has been removed from a plant-origin saponin (glycoside).

The method for obtaining Ingredient (B) is not particularly limited and may be appropriately selected depending on the intended purpose. Examples thereof include a method in which Ingredient (B) is extracted from plants, a method in which Ingredient (B) is obtained through synthesis, and a method in which commercially available products of Ingredient (B) are used.

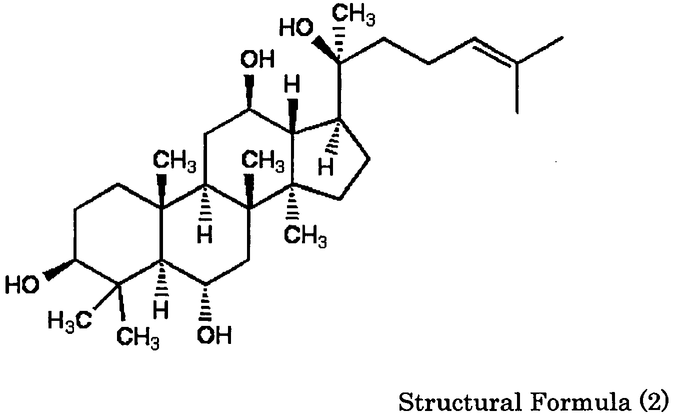

### <Ingredient (C)>

Panaxadiol (PD) (Ingredient (C)) is a compound having a structure expressed by the following Structural Formula (3).

Panaxadiol (PD) is a compound belonging to dammarane-type triterpenes. It is an aglycon formed after the sugar moiety has been removed from a plant-origin saponin (glycoside) and then the side chain has been ring-closed.

The method for obtaining Ingredient (C) is not particularly limited and may be appropriately selected depending on the intended purpose. Examples thereof include a method in which Ingredient (C) is extracted from plants, a method in which Ingredient (C) is obtained through synthesis, and a method in which commercially available products of Ingredient (C) are used.

Incorporation of Ingredient (C) into the composition can advantageously stabilize Ingredient (D).

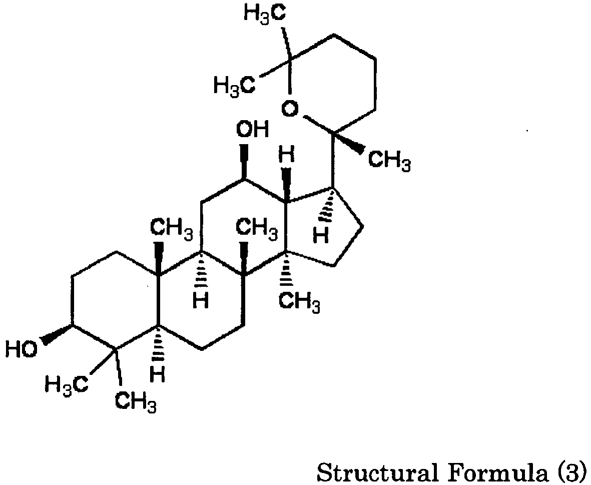

### <Ingredient (D)>

Protopanaxadiol (PPD) (Ingredient (D)) is a compound having a structure expressed by the following Structural Formula (4).

Protopanaxadiol (PPD) is a compound belonging to dammarane-type triterpenes. It is an aglycon formed after the sugar moiety has been removed from a plant-origin saponin (glycoside).

The method for obtaining Ingredient (D) is not particularly limited and may be appropriately selected depending on the intended purpose. Examples thereof include a method in which Ingredient (D) is extracted from plants, a method in which Ingredient (D) is obtained through synthesis, and a method in which commercially available products of Ingredient (D) are used.

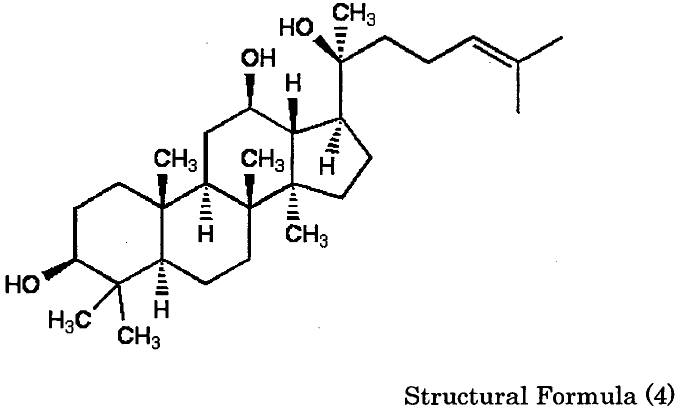

### <Amounts>

The amounts of Ingredients (A), (B), (C) and (D) contained in the composition are not particularly limited and may be appropriately selected depending on the intended purpose so long as the effects of the present invention are not impeded.

In the composition, Ingredient (A) is incorporated for stabilizing Ingredient (B) and Ingredient (C) is incorporated for stabilizing Ingredient (D). The composition may be a composition containing Ingredients (A) and (B) only among Ingredients (A), (B), (C) and (D), or may be a composition containing Ingredients (C) and (D) only among Ingredients (A), (B), (C) and (D). The composition may be a mixture of Ingredients (A) and (B) itself, or a mixture of Ingredients (C) and (D) itself.

### -Ratio by mass of Ingredient (A) to Ingredient (B)-

The ratio by mass of Ingredient (A) to Ingredient (B); i.e., (A)/(B) is not particularly limited, so long as it is 1 or greater, and may be appropriately selected depending on the intended purpose. The ratio (A)/(B) is preferably 9 or greater, more preferably 19 or greater. When the ratio by mass of Ingredient (A) to Ingredient (B) falls within the above preferred range, the stability of protopanaxatriol (PPT) increases.

Notably, having the "stability" means that when Ingredient (B) (protopanaxatriol (PPT)) is in the form of powder, the residual rate of Ingredient (B) is 90% by mass or more after the composition is left to stand still for one month under conditions of 40°C and relative humidity 75%.

Also, having the "stability" means that when Ingredient (B) (protopanaxatriol (PPT)) is in the form of ethanol solution, the residual rate of Ingredient (B) is 90% by mass or more after the composition is left to stand still for one month under conditions of 40°C and relative humidity 75%.

### -Ratio by mass of Ingredient (C) to Ingredient (D)-

The ratio by mass of Ingredient (C) to Ingredient (D); i.e., (C)/(D) is not particularly limited, so long as it is 1 or greater, and may be appropriately selected depending on the intended purpose. The ratio (C)/(D) is preferably 9 or greater, more preferably 19 or greater. When the ratio by mass of Ingredient (C) to Ingredient (D) falls within the above preferred range, the stability of protopanaxadiol (PPD) increases.

Notably, having the "stability" means that when Ingredient (D) (protopanaxadiol (PPD)) is in the form of powder, the residual rate of Ingredient (D) is 90% by mass or more after the composition has been left to stand still for one month under conditions of 40°C and relative humidity 75%.

Also, having the "stability" means that when Ingredient (D) (protopanaxadiol (PPD)) is in the form of ethanol solution, the residual rate of Ingredient (D) is 90% by mass or more after the composition has been left to stand still for one month under conditions of 40°C and relative humidity 75%.

### <Other ingredients>

The other ingredients are not particularly limited and may be appropriately selected depending on the intended purpose. Examples thereof include supplemental materials or additives commonly used for the production of foods or beverages.

The supplemental materials or additives are not particularly limited and may be appropriately selected depending on the intended purpose. Examples thereof include glucose, fructose, sucrose, maltose, sorbitol, stevioside, rubusoside, corn syrup, lactose, citric acid, tartaric acid, malic acid, succinic acid, lactic acid, L-ascorbic acid, dl-α-tocopherol, sodium erythorbate, glycerin, propylene glycol, glycerin fatty acid esters, polyglycerin fatty acid esters, sucrose fatty acid esters, sorbitan fatty acid esters, gum arabic, carrageenan, casein, gelatin, pectin, agar, B vitamins, nicotinic-acid amide, calcium pantothenate, amino acids, calcium salts, dyes, perfumes and preservatives.

The amount of the other ingredients contained in the composition is not particularly limited and may be appropriately selected depending on the intended purpose.

### <Use>

The composition is used in a food or beverage, in dietary supplements or pharmaceutical drugs, which can be given orally.

Here, "food or beverage" refers to those which are less harmful to human health and which are given orally or through the gastrointestinal tract in the ordinary social life. They are not limited to foods, drugs and quasi drugs within the administrative boundaries, but include a wide variety of orally-given common foods, healthy foods, health-promoting foods, quasi drugs and drugs.

The composition may be a food or beverage itself, or may be incorporated into a food or beverage.

The amount of the composition incorporated into a food or beverage is not particularly limited and may be appropriately selected depending on the type of the food or beverage so long as the effects of the present invention are not impeded.

### <Food or beverage>

The food or beverage is not particularly limited and may be appropriately selected depending on the intended purpose so long as it contains at least one of a mixture of Ingredients (A) and (B) and a mixture of Ingredients (C) and (D). Examples thereof include beverages such as refreshing beverages, carbonated beverages, energy beverages, fruit beverages and lactic beverages; frozen desserts such as ice cream, ice sherbet and ice shavings; noodles such as buckwheat noodles, wheat noodles, vermicelli, coats of Chinese dumplings, coats of pork dumplings, Chinese noodles and instant noodles; snacks such as candies, gum, chocolate, tabletted snacks, munches, biscuits, jelly, jam, cream, baked confectionery and bread; marine products such as crab, salmon, Japanese littleneck, tuna, sardine, shrimps, prawns, bonito, mackerel, whale, oyster, saury, squid, bloody clam, scallop, abalone, sea chestnut, salmon caviar and *Sulculus diversicolor supertexta*; marine/livestock processed foods such as fish minced and steamed, ham and sausage; dairy products such as processed milk and fermented milk; fats and oils or processed foods thereof such as salad oil, *Tempura* oil, margarine, mayonnaise, shortening, whip cream and dressing; seasonings such as sauce and basting; retort pouch foods such as curry, stew, *Oyako-don* (a bowl of rice topped with boiled chicken and eggs), rice porridge, *Zosui* (rice soup), *Chuka-don* (a bowl of rice with a chop-suey-like mixture on it), *Katsu-don* (a rice bowl with pork cutlets), *Ten-don* (a *tempura* rice bowl), *Una-don* (an eel rice bowl), *hayashi rice* (hashed beef with rice), *Oden* (a dish containing several ingredients such as boiled eggs and radish), mapo doufu, *Gyu-don* (a beef rice bowl), meat sauce, egg soup, rice omelet, Chinese dumplings, pork dumplings, hamburger steak and meat balls; healthy foods in various forms and dietary supplements; and pharmaceutical drugs and quasi drugs such as tablets, capsules, drinkable preparations, jelly preparations and troches.

### <Dosage form>

The dosage form of the food or beverage is not particularly limited and may be appropriately selected depending on the intended purpose. Examples thereof include an oral solid preparation, an oral semisolid preparation and an oral liquid preparation.

Ingredients (B) and (D) are generally poor in stability and thus are not suitable to use in a system containing a large amount of water and a low-pH system. However, Ingredients (B) and (D) in the composition for use or used according to the present invention are stable even in a system containing a large amount of water and a low-pH system, and thus may be formed into an oral semisolid preparation and an oral liquid preparation.

### -Oral solid preparation-

The oral solid preparation is not particularly limited and may be appropriately selected depending on the intended purpose. Examples thereof include a tablet, a coated tablet, granules, powder and a capsule.

The method for producing the oral solid preparation is not particularly limited and may be a routine method. For example, the oral solid preparation can be produced by adding an excipient and, if necessary, various additives to at least one of a mixture of Ingredients (A) and (B) and a mixture of Ingredients (C) and (D). Here, the excipient is not particularly limited and may be appropriately selected depending on the intended purpose. Examples of the excipient include lactose, sucrose, sodium chloride, glucose, starch, calcium carbonate, kaolin, microcrystalline cellulose and silicic acid. The additives are not particularly limited and may be appropriately selected depending on the intended purpose. Examples of the additives include a binding agent, a disintegrating agent, a lubricating agent, a coloring agent and a sweetening/flavoring agent.

The binding agent is not particularly limited and may be appropriately selected depending on the intended purpose. Examples of the binding agent include water, ethanol, propanol, simple syrup, glucose liquid, starch liquid, gelatine liquid, carboxymethylcellulose, hydroxypropylcellulose, hydroxypropylstarch, methylcellulose, ethylcellulose, shellac, calcium phosphate and polyvinylpyrrolidone.

The disintegrating agent is not particularly limited and may be appropriately selected depending on the intended purpose. Examples of the disintegrating agent include dry starch, sodium alginate, powdered agar, sodium hydrogencarbonate, calcium carbonate, sodium lauryl sulfate, monoglyceride stearate and lactose.

The lubricating agent is not particularly limited and may be appropriately selected depending on the intended purpose. Examples of the lubricating agent include purified talc, stearic acid salts, borax and polyethylene glycol.

The coloring agent is not particularly limited and may be appropriately selected depending on the intended purpose. Examples of the coloring agent include titanium oxide and iron oxide.

The sweetening/flavoring agent is not particularly limited and may be appropriately selected depending on the intended purpose. Examples of the sweetening/flavoring agent include sucrose, orange peel, citric acid and tartaric acid.

### -Oral semisolid preparation-

The oral semisolid preparation is not particularly limited and may be appropriately selected depending on the intended purpose so long as it has intermediate properties between a liquid preparation and a solid preparation. Examples thereof include electuary, a chewing gum preparation, a whip preparation and a jelly preparation.

The method for producing the oral semisolid preparation is not particularly limited and may be a routine method. For example, the oral semisolid preparation can be produced by adding a gelling agent, a thickening agent and a stabilizing agent to at least one of a mixture of Ingredients (A) and (B) and a mixture of Ingredients (C) and (D).

The gelling agent is not particularly limited and may be appropriately selected depending on the intended purpose. Examples thereof include agar, gelatin, starch and gellan.

The thickening agent is not particularly limited and may be appropriately selected depending on the intended purpose. Examples thereof include xanthan, carrageenan, locust, guar, tamarind and pectin.

The stabilizing agent is not particularly limited and may be appropriately selected depending on the intended purpose. Examples thereof include tragacanth, gum arabic and gum ghatti.

### -Oral liquid preparation-

The oral liquid preparation is not particularly limited and may be appropriately selected depending on the intended purpose. Examples thereof include an internal liquid preparation, syrup and elixir.

The method for producing the oral liquid preparation is not particularly limited and may be a routine method. For example, the oral liquid preparation can be produced by adding an additive to at least one of a mixture of Ingredients (A) and (B) and a mixture of Ingredients (C) and (D).

The additive is not particularly limited and may be appropriately selected depending on the intended purpose. Examples thereof include a sweetening/flavoring agent, a buffer and a stabilizing agent.

The sweetening/flavoring agent is not particularly limited and may be appropriately selected depending on the intended purpose. Examples thereof include sucrose, orange peel, citric acid and tartaric acid.

The buffer is not particularly limited and may be appropriately selected depending on the intended purpose. Examples thereof include sodium citrate.

The stabilizing agent is not particularly limited and may be appropriately selected depending on the intended purpose. Examples thereof include tragacanth, gum arabic and gelatin.

### <Intake>

The method, amount, time and target of intake of the composition are not particularly limited and may be appropriately selected depending on the intended purpose.

The amount of intake thereof is not particularly limited and may be appropriately determined considering various factors of target individuals such as their age, body weight, constitution, symptoms and concomitant use of a drug containing other active ingredients.

Regarding animal species that can be targets of intake thereof, the composition is suitably applied to human. However, so long as the effects of the composition can be obtained, the composition may also be applied to non-human animals such as mice, rats, hamsters, birds, dogs, cats, sheep, goats, bovine, pigs and monkeys.

### <Application>

The composition are used for a food or beverage, in dietary supplements or in pharmaceutical drugs having physiological activities such as an anti-cancer effect, an anti-inflammatory effect and a sugar metabolism-regulating effect.

### Examples

The present invention will next be described in more detail by way of Examples, which should not be construed as limiting the present invention thereto. (Comparative Examples 1 and 2: Stabilities of PPT (Ingredient (B)) and PPD (Ingredient (D)))

### <Method>

5 mg of protopanaxatriol (PPT) powder (Ingredient (B)) (product of LKT Laboratories Inc.) (Comparative Example 1) and 5 mg of protopanaxadiol (PPD) powder (Ingredient (D)) (product of LKT Laboratories Inc.) (Comparative Example 2) were separately added to brown bottles with screw caps and left to stand still for 3 weeks under conditions of 40°C and relative humidity 75%. After that, about 1 mg of each of the protopanaxatriol (PPT) powder (Ingredient (B)) and the protopanaxadiol (PPD) powder (Ingredient (D)) was dissolved in 1 mL of ethanol for high-performance liquid chromatography (purity: 99.5% by mass) (product of Wako Pure Chemical Industries, Ltd.) and analyzed through high-performance liquid chromatography under the following conditions.

### -Analysis conditions-

Apparatus: 1200 series high-performance liquid chromatograph (product of Agilent Technologies, Ltd.)
Column: TSKgel ODS-80Ts, inner diameter: 4.6 mm, length: 15 cm (product of TOSOH CORPORATION, Ltd.)
Column temperature: 40°C
Eluent: water : acetonitrile = 50 : 50 (for analysis of PPT)
   water: acetonitrile = 30 : 70 (for analysis of PPD)
Measurement wavelength: 196 nm
Flow rate: 1 mL/min
Injection amount: 10 µL

### <Results>

As a result of the above analysis, neither the protopanaxatriol (PPT) powder (Ingredient (B)) (Comparative Example 1) nor the protopanaxadiol (PPD) powder (Ingredient (D)) (Comparative Example 2) was detected. These results indicate that the protopanaxatriol (PPT) powder (Ingredient (B)) and the protopanaxadiol (PPD) powder (Ingredient (D)) are very poor in stability when they are left to stand still alone.

### (Examples 1 to 3 and Comparative Examples 3 to 7: Mixing ratio of PT (Ingredient (A)) and PPT (Ingredient (B)) used in combination and stability of PPT (Ingredient (B)))

Panaxatriol (PT) powder (Ingredient (A)) (product of LKT Laboratories Inc.) and protopanaxatriol (PPT) powder (Ingredient (B)) (product of LKT Laboratories Inc.) were mixed together at mixing ratios as shown in Table 1 so that the total mass thereof was 10 mg. Each of the resultant mixtures was added to a brown bottle with a screw cap and left to stand still for one month under conditions of 40°C and relative humidity 75%. After that, about 10 mg of the mixture of the panaxatriol (PT) powder (Ingredient (A)) and the protopanaxatriol (PPT) powder (Ingredient (B)), which had been left to stand still under the above conditions, was dissolved in 1 mL of ethanol for high-performance liquid chromatography (purity: 99.5% by mass) (product of Wako Pure Chemical Industries, Ltd.). The resultant ethanol solution was analyzed through high-performance liquid chromatography under the same analysis conditions as in Comparative Examples 1 and 2, to thereby quantify the amounts of panaxatriol (PT) (Ingredient (A)) and protopanaxatriol (PPT) (Ingredient (B)). The results are shown in Table 1.

**Table 1**

| | Comparative Examples | | | | | Examples | | |
|---|---|---|---|---|---|---|---|---|
| | 3 | 4 | 5 | 6 | 7 | 1 | 2 | 3 |
| (A) Amount of PT (mg) | 0.1 | 1 | 2 | 3 | 4 | 5 | 9 | 9.5 |
| (B) Amount of PPT (mg) | 9.9 | 9 | 8 | 7 | 6 | 5 | 1 | 0.5 |
| (A) PT/(B) PPT (ratio by mass) | 1/99 | 10/90 | 20/80 | 30/70 | 40/60 | 50/50 | 90/10 | 95/5 |
| | 0.01 | 0.11 | 0.25 | 0.43 | 0.67 | 1 | 9 | 19 |
| (A) Residual rate of PT (% by mass) | 98 | 98 | 97 | 100 | 101 | 99 | 101 | 98 |
| (B) Residual rate of PPT (% by mass) | 0 | 4 | 11 | 32 | 72 | 92 | 95 | 100 |

### <Results>

As is clear from Table 1, it is confirmed that the ratio by mass of (A) PT/(B) PPT is preferably adjusted to 50/50 (= 1) or greater, more preferably 90/10 (= 9) or greater, still more preferably 95/5 (= 19) or greater, in order to keep the amount of PPT for one month under conditions of 40°C and relative humidity 75%.

### (Examples 4 to 6 and Comparative Examples 8 to 12: Mixing ratio of PD (Ingredient (C)) and PPD (Ingredient (D)) used in combination and stability of PPD (Ingredient (D)))

Panaxadiol (PD) powder (Ingredient (C)) (product of LKT Laboratories Inc.) and protopanaxadiol (PPD) powder (Ingredient (D)) (product of LKT Laboratories Inc.) were mixed together at mixing ratios as shown in Table 2 so that the total mass thereof was 10 mg. Each of the resultant mixtures was added to a brown bottle with a screw cap and left to stand still for one month under conditions of 40°C and relative humidity 75%. After that, about 10 mg of the mixture of the panaxadiol (PD) powder (Ingredient (C)) and the protopanaxadiol (PPD) powder (Ingredient (D)), which had been left to stand still under the above conditions, was dissolved in 1 mL of ethanol for high-performance liquid chromatography (purity: 99.5% by mass) (product of Wako Pure Chemical Industries, Ltd.). The resultant ethanol solution was analyzed through high-performance liquid chromatography under the same analysis conditions as in Comparative Examples 1 and 2, to thereby quantify the amounts of panaxadiol (PD) (Ingredient (C)) and protopanaxadiol (PPD) (Ingredient (D)). The results are shown in Table 2.

**Table 2**

| | Comparative Examples | | | | | Examples | | |
|---|---|---|---|---|---|---|---|---|
| | 8 | 9 | 10 | 11 | 12 | 4 | 5 | 6 |
| (C) Amount of PD (mg) | 0.1 | 1 | 2 | 3 | 4 | 5 | 9 | 9.5 |
| (D) Amount of PPD (mg) | 9.9 | 9 | 8 | 7 | 6 | 5 | 1 | 0.5 |
| (C) PD/(D) PPD (ratio by mass) | 1/99 | 10/90 | 20/80 | 30/70 | 40/60 | 50/50 | 90/10 | 95/5 |
| | 0.01 | 0.11 | 0.25 | 0.43 | 0.67 | 1 | 9 | 19 |
| (C) Residual rate of PD (% by mass) | 98 | 99 | 100 | 100 | 99 | 101 | 100 | 101 |
| (D) Residual rate of PPD (% by mass) | 0 | 6 | 14 | 41 | 75 | 93 | 95 | 100 |

### <Results>

As is clear from Table 2, it is confirmed that the ratio by mass of (C) PD/(D) PPD is preferably adjusted to 50/50 (= 1) or greater, more preferably 90/10 (= 9) or greater, still more preferably 95/5 (= 19) or greater, in order to keep the amount of PPD for one month under conditions of 40°C and relative humidity 75%.

### (Examples 7 to 15 and Comparative Examples 13 to 27: Stability of PPT (Ingredient (B)) used in combination with PT (Ingredient (A)) in an ethanol solution)

### <Method>

10 mg of panaxatriol (PT) (Ingredient (A)) (product of LKT Laboratories, Inc.) and 10 mg of protopanaxatriol (PPT) (Ingredient (B)) (product of LKT Laboratories, Inc.) were each dissolved in 0.5 mL of ethanol. The pH of each of the solutions was adjusted to 3.5, 6.8 or 8.3 with 5% by mass hydrochloric acid or 1M aqueous sodium hydroxide. The PT ethanol solution and the PPT ethanol solution, whose pH had been adjusted to each of these values, were mixed together with appropriately diluted with ethanol, to thereby prepare ethanol solutions having PT and PPT concentrations shown in Tables 3 to 5. Each of the thus-prepared ethanol solutions was added to a brown bottle with a screw cap and left to stand still for one month under conditions of 40°C and relative humidity 75%. After that, the panaxatriol (PT) (Ingredient (A)) ethanol solution and the protopanaxatriol (PPT) (Ingredient (B)) ethanol solution, which had been left to stand still under the above conditions, were 10-fold diluted with ethanol. The diluted ethanol solutions were analyzed through high-performance liquid chromatography under the same analysis conditions as in Comparative Examples 1 and 2, to thereby analyze the amount of the protopanaxatriol (PPT) (Ingredient (B)). The obtained value was used to calculate a residual rate (% by mass) of the PPT relative to the initial amount thereof. The results are shown in Tables 3 to 5.

**Table 3**

| | Comparative Examples | | | | | Examples | | |
|---|---|---|---|---|---|---|---|---|
| | 13 | 14 | 15 | 16 | 17 | 7 | 8 | 9 |
| pH | 3.5 | 3.5 | 3.5 | 3.5 | 3.5 | 3.5 | 3.5 | 3.5 |
| (A) Amount of PT (% by mass) | 0.01 | 0.1 | 0.2 | 0.3 | 0.4 | 0.5 | 0.9 | 0.95 |
| (B) Amount of PPT (% by mass) | 0.99 | 0.9 | 0.8 | 0.7 | 0.6 | 0.5 | 0.1 | 0.05 |
| (A) PT/(B) PPT (ratio by mass) | 1/99 | 10/90 | 20/80 | 30/70 | 40/60 | 50/50 | 90/10 | 95/5 |
| | 0.01 | 0.11 | 0.25 | 0.43 | 0.67 | 1 | 9 | 19 |
| (B) Residual rate of PPT (% by mass) | 0 | 0 | 0.4 | 25.8 | 58.6 | 90.2 | 91.4 | 94.7 |

**Table 4**

| | Comparative Examples | | | | | Examples | | |
|---|---|---|---|---|---|---|---|---|
| | 18 | 19 | 20 | 21 | 22 | 10 | 11 | 12 |
| pH | 6.8 | 6.8 | 6.8 | 6.8 | 6.8 | 6.8 | 6.8 | 6.8 |
| (A) Amount of PT (% by mass) | 0.01 | 0.1 | 0.2 | 0.3 | 0.4 | 0.5 | 0.9 | 0.95 |
| (B) Amount of PPT (% by mass) | 0.99 | 0.9 | 0.8 | 0.7 | 0.6 | 0.5 | 0.1 | 0.05 |
| (A) PT/(B) PPT (ratio by mass) | 1/99 | 10/90 | 20/80 | 30/70 | 40/60 | 50/50 | 90/10 | 95/5 |
| | 0.01 | 0.11 | 0.25 | 0.43 | 0.67 | 1 | 9 | 19 |
| (B) Residual rate of PPT (% by mass) | 0 | 0.1 | 0.6 | 27.8 | 60.2 | 91.6 | 94.2 | 98.7 |

**Table 5**

| | Comparative Examples | | | | | Examples | | |
|---|---|---|---|---|---|---|---|---|
| | 23 | 24 | 25 | 26 | 27 | 13 | 14 | 15 |
| pH | 8.3 | 8.3 | 8.3 | 8.3 | 8.3 | 8.3 | 8.3 | 8.3 |
| (A) Amount of PT (% by mass) | 0.01 | 0.1 | 0.2 | 0.3 | 0.4 | 0.5 | 0.9 | 0.95 |
| (B) Amount of PPT (% by mass) | 0.99 | 0.9 | 0.8 | 0.7 | 0.6 | 0.5 | 0.1 | 0.05 |
| (A) PT/(B) PPT (ratio by mass) | 1/99 | 10/90 | 20/80 | 30/70 | 40/60 | 50/50 | 90/10 | 95/5 |
| | 0.01 | 0.11 | 0.25 | 0.43 | 0.67 | 1 | 9 | 19 |
| (B) Residual rate of PPT (% by mass) | 0 | 0.8 | 1.4 | 30.9 | 62.8 | 91.4 | 93.4 | 97.1 |

### <Results>

As is clear from Tables 3 to 5, when the ratio by mass of (A) PT/(B) PPT was smaller than 50/50 (= 1), the stability of the PPT (Ingredient (B)) was poor (Comparative Examples 13 to 27). Especially when the pH of the ethanol solutions was lower, the residual rate of the PPT (Ingredient (B)) was lower (Comparative Examples 13 to 17). Meanwhile, when the ratio by mass of (A) PT/(B) PPT was 50/50 (= 1) or greater, the residual rate of the PPT was 90% by mass or greater in all the ethanol solutions regardless of the pH thereof (Examples 7 to 15). It was confirmed that the stability of the PPT (Ingredient (B)) was kept even at the low pH (Examples 7 to 9).

These results confirm that the ratio by mass of (A) PT/(B) PPT is preferably adjusted to 50/50 (= 1) or greater, more preferably 90/10 (= 9) or greater, still more preferably 95/5 (= 19) or greater, in order to keep the amount of PPT for one month. (Examples 16 to 24 and Comparative Examples 28 to 42: Stability of PPD (Ingredient (D)) used in combination with PD (Ingredient (C)) in an ethanol solution)

### <Method>

10 mg of panaxadiol (PD) (Ingredient (C)) (product of LKT Laboratories, Inc.) and 10 mg of protopanaxadiol (PPD) (Ingredient (D)) (product of LKT Laboratories, Inc.) were each dissolved in 0.5 mL of ethanol. The pH of each of the solutions was adjusted to 3.5, 6.8 or 8.3 with 5% by mass hydrochloric acid or 1M aqueous sodium hydroxide. The PD ethanol solution and the PPD ethanol solution, whose pH had been adjusted to each of these values, were mixed together with appropriately diluted with ethanol, to thereby prepare ethanol solutions having PD and PPD concentrations shown in Tables 6 to 8. Each of the thus-prepared ethanol solutions was added to a brown bottle with a screw cap and left to stand still for one month under conditions of 40°C and relative humidity 75%. After that, the protopanaxadiol (PPD) (Ingredient (D)) ethanol solution and the panaxadiol (PD) (Ingredient (C)) ethanol solution, which had been left to stand still under the above conditions, were 10-fold diluted with ethanol. The diluted ethanol solutions were analyzed through high-performance liquid chromatography under the same analysis conditions as in Comparative Examples 1 and 2, to thereby analyze the amount of the protopanaxadiol (PPD) (Ingredient (D)). The obtained value was used to calculate a residual rate (% by mass) of the PPD relative to the initial amount thereof. The results are shown in Tables 6 to 8.

**Table 6**

| | Comparative Examples | | | | | Examples | | |
|---|---|---|---|---|---|---|---|---|
| | 28 | 29 | 30 | 31 | 32 | 16 | 17 | 18 |
| pH | 3.5 | 3.5 | 3.5 | 3.5 | 3.5 | 3.5 | 3.5 | 3.5 |
| (C) Amount of PD (% by mass) | 0.01 | 0.1 | 0.2 | 0.3 | 0.4 | 0.5 | 0.9 | 0.95 |
| (D) Amount of PPD (% by mass) | 0.99 | 0.9 | 0.8 | 0.7 | 0.6 | 0.5 | 0.1 | 0.05 |
| (C) PD/(D) PPD (ratio by mass) | 1/99 | 10/90 | 20/80 | 30/70 | 40/60 | 50/50 | 90/10 | 95/5 |
| | 0.01 | 0.11 | 0.25 | 0.43 | 0.67 | 1 | 9 | 19 |
| (D) Residual rate of PPD (% by mass) | 0 | 0 | 0.8 | 23.5 | 58.5 | 90.1 | 90.8 | 94.8 |

**Table 7**

| | Comparative Examples | | | | | Examples | | |
|---|---|---|---|---|---|---|---|---|
| | 33 | 34 | 35 | 36 | 37 | 19 | 20 | 21 |
| pH | 6.8 | 6.8 | 6.8 | 6.8 | 6.8 | 6.8 | 6.8 | 6.8 |
| (C) Amount of PD (% by mass) | 0.01 | 0.1 | 0.2 | 0.3 | 0.4 | 0.5 | 0.9 | 0.95 |
| (D) Amount of PPD (% by mass) | 0.99 | 0.9 | 0.8 | 0.7 | 0.6 | 0.5 | 0.1 | 0.05 |
| (C) PD/(D) PPD (ratio by mass) | 1/99 | 10/90 | 20/80 | 30/70 | 40/60 | 50/50 | 90/10 | 95/5 |
| | 0.01 | 0.11 | 0.25 | 0.43 | 0.67 | 1 | 9 | 19 |
| (D) Residual rate of PPD (% by mass) | 0.5 | 3.0 | 6.3 | 40.6 | 69.5 | 91.2 | 95.7 | 99.8 |

**Table 8**

| | Comparative Examples | | | | | Examples | | |
|---|---|---|---|---|---|---|---|---|
| | 38 | 39 | 40 | 41 | 42 | 22 | 23 | 24 |
| pH | 8.3 | 8.3 | 8.3 | 8.3 | 8.3 | 8.3 | 8.3 | 8.3 |
| (C) Amount of PD (% by mass) | 0.01 | 0.1 | 0.2 | 0.3 | 0.4 | 0.5 | 0.9 | 0.95 |
| (D) Amount of PPD (% by mass) | 0.99 | 0.9 | 0.8 | 0.7 | 0.6 | 0.5 | 0.1 | 0.05 |
| (C) PD/(D) PPD (ratio by mass) | 1/99 | 10/90 | 20/80 | 30/70 | 40/60 | 50/50 | 90/10 | 95/5 |
| | 0.01 | 0.11 | 0.25 | 0.43 | 0.67 | 1 | 9 | 19 |
| (D) Residual rate of PPD (% by mass) | 0 | 0.5 | 1.8 | 43.5 | 74.3 | 90.8 | 94.7 | 98.8 |

### <Results>

As is clear from Tables 6 to 8, when the ratio by mass of (C) PD/(D) PPD was smaller than 50/50 (= 1), the stability of the PPD (Ingredient (D)) was poor (Comparative Examples 28 to 42). Especially when the pH of the ethanol solutions was lower, the residual rate of the PPD (Ingredient (D)) was lower (Comparative Examples 28 to 32). Meanwhile, when the ratio by mass of (C) PD/(D) PPD was 50/50 (= 1) or greater, the residual rate of the PPD was 90% by mass or greater in all the ethanol solutions regardless of the pH thereof (Examples 16 to 24). It was confirmed that the stability of the PPD (Ingredient (D)) was kept even at the low pH (Examples 16 to 18).

These results confirm that the ratio by mass of (C) PD/(D) PPD is preferably adjusted to 50/50 (= 1) or greater, more preferably 90/10 (= 9) or greater, still more preferably 95/5 (= 19) or greater, in order to keep the amount of PPD for one month.

Although protopanaxatriol (PPT) (Ingredient (B)) and protopanaxadiol (PPD) (Ingredient (D)) have excellent physiological activities such as an anti-cancer effect, an anti-inflammatory effect and a sugar metabolism-regulating effect, their stability is poor to thereby make it difficult to provide a composition containing a large amount of PPT (Ingredient (B)) and PPD (Ingredient (D)). However, as is understood from the results of Examples 1 to 24, it is possible to provide a composition containing a large amount of PPT (Ingredient (B)) and PPD (Ingredient (D)).

### Industrial Applicability

The composition of the present invention contains a large amount of at least one of protopanaxatriol (PPT) and protopanaxadiol (PPD) and also has high stability. Thus, it can be used as a composition capable of stably exhibiting excellent physiological activities such as an anti-cancer effect, an anti-inflammatory effect and a sugar metabolism-regulating effect. The composition is also suitably used in or as a food or beverage, in dietary supplements or in pharmaceutical drugs.

## Claims

1. A composition for medical use in pharmaceutical drugs comprising:
at least one mixture selected from the group consisting of a mixture of (A) panaxatriol and (B) protopanaxatriol and a mixture of (C) panaxadiol and (D) protopanaxadiol,
wherein a ratio (A)/(B) of a mass of the (A) panaxatriol to a mass of the (B) protopanaxatriol is 1 or greater, and a ratio (C)/(D) of a mass of the (C) panaxadiol to a mass of the (D) protopanaxadiol is 1 or greater.

2. Use of a composition comprising:
at least one mixture selected from the group consisting of a mixture of (A) panaxatriol and (B) protopanaxatriol and a mixture of (C) panaxadiol and (D) protopanaxadiol,
wherein a ratio (A)/(B) of a mass of the A) panaxatriol to a mass of the (B) protopanaxatriol is 1 or greater, and a ratio (C)/(D) of a mass of the (C) panaxadiol to a mass of the (D) protopanaxadiol is 1 or greater for the preparation of food or beverage, dietary supplements or of pharmaceutical drugs.

3. Use of a composition according to claim 2, wherein the composition is a food or beverage.

4. Use of a composition according to claim 2, wherein the dosage form is an oral solid preparation, an oral semisolid preparation or an oral liquid preparation.

5. Use of a composition according to claim 4, wherein the composition is an oral solid preparation further comprising an excipient, binding agent, lubricating agent, a disintegrating agent, coloring agent, and/or sweeten ing/flavoring agent.

## Patentansprüche

1. Eine Zusammensetzung zur medizinischen Verwendung in Pharmazeutika, umfassend:
mindestens eine Mischung ausgewählt aus der Gruppe, bestehend aus einer Mischung von (A) Panaxatriol und (B) Protopanaxatriol und einer Mischung aus (C) Panaxadiol und (D) Protopanaxadiol,
wobei das Verhältnis (A)/(B) einer Masse von (A) Panaxatriol zu einer Masse von (B) Protopanaxatriol 1 oder größer ist, und das Verhältnis (C)/(D) einer Masse von (C) Panaxadiol zu einer Masse von (D) Protopanaxadiol 1 oder größer ist.

2. Verwendung einer Zusammensetzung, umfassend:
mindestens eine Mischung ausgewählt aus der Gruppe, bestehend aus einer Mischung von (A) Panaxatriol und (B) Protopanaxatriol und einer Mischung aus (C) Panaxadiol und (D) Protopanaxadiol,
wobei das Verhältnis (A)/(B) einer Masse von (A) Panaxatriol zu einer Masse von (B) Protopanaxatriol 1 oder größer ist, und das Verhältnis (C)/(D) einer Masse von (C) Panaxadiol zu einer Masse von (D) Protopanaxadiol 1 oder größer ist, für die Herstellung von Nahrungsmittel oder Getränk, Nahrungsergänzungsmitteln oder von Pharmazeutika.

3. Verwendung einer Zusammensetzung gemäß Anspruch 2, wobei die Zusammensetzung ein Nahrungsmittel oder Getränk ist.

4. Verwendung einer Zusammensetzung gemäß Anspruch 2, wobei die Dosierungsform eine orale feste Zubereitung, eine orale halbfeste Zubereitung oder eine orale flüssige Zubereitung ist.

5. Verwendung einer Zusammensetzung gemäß Anspruch 4, wobei die Zusammensetzung eine orale feste Zubereitung ist, ferner umfassend einen Hilfsstoff, ein Bindemittel, ein Gleitmittel, ein Auflösungsmittel, ein Färbemittel, und/oder Süßungs-/Geschmacksmittel. I.

## Revendications

1. Composition à usage médical dans des médicaments pharmaceutiques comprenant :
au moins un mélange choisi dans le groupe constitué d'un mélange de (A) panaxatriol et (B) protopanaxatriol et d'un mélange de (C) panaxadiol et (D) protopanaxadiol,
dans laquelle un rapport (A)/(B) de la masse du (A) panaxatriol à la masse du (B) protopanaxatriol est supérieur ou égal à 1, et un rapport (C)/(D) de la masse du (C) panaxadiol à la masse du (D) protopanaxadiol est supérieur ou égal à 1.

2. Utilisation d'une composition comprenant :
au moins un mélange choisi dans le groupe constitué d'un mélange de (A) panaxatriol et (B) protopanaxatriol et d'un mélange de (C) panaxadiol et (D) protopanaxadiol,
dans laquelle un rapport (A)/(B) de la masse du A) panaxatriol à la masse du (B) protopanaxatriol est supérieur ou égal à 1, et un rapport (C)/(D) de la masse du (C) panaxadiol à la masse du (D) protopanaxadiol est supérieur ou égal à 1
pour la préparation d'un aliment ou d'une boisson, de compléments alimentaires ou de médicaments pharmaceutiques.

3. Utilisation d'une composition selon la revendication 2, dans laquelle la composition est un aliment ou une boisson.

4. Utilisation d'une composition selon la revendication 2, dans laquelle la forme pharmaceutique est une préparation solide orale, une préparation semi-solide orale ou une préparation liquide orale.

5. Utilisation d'une composition selon la revendication 4, dans laquelle la composition est une préparation solide orale comprenant en outre un excipient, un agent liant, un agent lubrifiant, un agent de désintégration, un colorant et/ou un édulcorant/arôme.
